# EUROPÄISCHE PATENTSCHRIFT

(11) **EP 3 625 352 B1**
(45) Veröffentlichungstag und Bekanntmachung des Hinweises auf die Patenterteilung: **24.01.2024**
(21) Anmeldenummer: 18725503.9
(22) Anmeldetag: 17.05.2018
(51) Int. Cl.: C12P 15/00, C12N 9/02, C12N 9/10, C12N 9/90

(54) **VERFAHREN ZUR FERMENTATIVEN DE NOVO SYNTHESE VON HARZSÄUREN**
METHOD FOR THE FERMENTATIVE DE NOVO SYNTHESIS OF RESIN ACIDS
PROCÉDÉ DE SYNTHÈSE DE NOVO FERMENTABLE D'ACIDES RÉSINIQUES

(30) Priorität: 19.05.2017 EP 17172021
(43) Veröffentlichungstag der Anmeldung: 25.03.2020
(73) Patentinhaber: Jowat SE, 32758 Detmold (DE)
(72) Erfinder: JACH, Guido, 53639 Königswinter (DE); SCHULLEHNER, Katrin, 51375 Leverkusen (DE); WELTERS, Peter, 41334 Nettetal (DE)
(74) Vertreter: dompatent von Kreisler Selting Werner - Partnerschaft von Patent- und Rechtsanwälten mbB
(86) Internationale Anmeldenummer: PCT/EP2018/062877
(87) Internationale Veröffentlichungsnummer: WO 2018/211002

(56) Entgegenhaltungen:
- WO-A1-2011/060044
- WO-A1-2015/197075
- WO-A1-2017/036495
- KATRIN GEISLER ET AL: "Modularity of Conifer Diterpene Resin Acid Biosynthesis: P450 Enzymes of Different CYP720B Clades Use Alternative Substrates and Converge on the Same Products", PLANT PHYSIOLOGY, Bd. 171, Nr. 1, 2. März 2016 (2016-03-02), Seiten 152-164, XP55484549, Rockville, Md, USA ISSN: 0032-0889, DOI: 10.1104/pp.16.00180
- B. HAMBERGER ET AL: "Evolution of Diterpene Metabolism: Sitka Spruce CYP720B4 Catalyzes Multiple Oxidations in Resin Acid Biosynthesis of Conifer Defense against Insects", PLANT PHYSIOLOGY, Bd. 157, Nr. 4, 12. Oktober 2011 (2011-10-12), Seiten 1677-1695, XP055119176, ISSN: 0032-0889, DOI: 10.1104/pp.111.185843
- Dae-Kyun Ro ET AL: "Loblolly pine abietadienol/abietadienal oxidase PtAO (CYP720B1) is a multifunctional, multisubstrate cytochrome P450 monooxygenase", PNAS, Bd. 102, Nr. 22 1. Januar 2005 (2005-01-01), 31. Mai 2005 (2005-05-31), Seiten 8060-8065, XP055215826, Gefunden im Internet: URL:http://www.pnas.org/content/102/22/806 0.full.pdf?with-ds=yes [gefunden am 2015-09-24]
- THIYAGARAJAN GNANASEKARAN ET AL: "Heterologous expression of the isopimaric acid pathway in Nicotiana benthamiana and the effect of N-terminal modifications of the involved cytochrome P450 enzyme", JOURNAL OF BIOLOGICAL ENGINEERING, Bd. 9, Nr. 1, 1. Dezember 2015 (2015-12-01), XP055413444, DOI: 10.1186/s13036-015-0022-z
- GONG HAI-YAN ET AL: "Diterpene synthases and their responsible cyclic natural products", NATURAL PRODUCTS AND BIOPROSPECTING, SPRINGER OPEN, GERMANY , Bd. 4, Nr. 2 1. April 2014 (2014-04-01), Seiten 59-72, XP002739034, ISSN: 2192-2209, DOI: 10.1007/S13659-014-0012-8 Gefunden im Internet: URL:http://rd.springer.com/article/10.1007 %2Fs13659-014-0012-8 [gefunden am 2014-04-18]
- KEELING C I ET AL: "Diterpene resin acids in conifers", PHYTOCHEMISTRY, PERGAMON PRESS, GB, Bd. 67, Nr. 22, 1. November 2006 (2006-11-01), Seiten 2415-2423, XP028059968, ISSN: 0031-9422, DOI: 10.1016/J.PHYTOCHEM.2006.08.019 [gefunden am 2006-11-01]

## Beschreibung

### Technisches Gebiet der Erfindung

Die vorliegende Erfindung betrifft rekombinante Bakterien zur fermentativen *de novo* Synthese von Harzsäuregemischen definierter Zusammensetzung, wobei die rekombinanten Bakterien die Enzymaktivitäten wenigstens einer Geranylgeranyl-Pyrophosphat-Synthase (GGPPS), wenigstens einer Isopentenyl-Pyrophosphat-Isomerase (IPI), wenigstens einer Diterpensynthase (diTPS), wenigstens einer Cytochrom-P450-Monooxygenase (CYP), und wenigstens einer Cytochrom-P450-Reduktase (CPR) aufweisen, die Enzyme exprimieren und die Harzsäuregemische definierter Zusammensetzung synthetisieren, wobei die Zusammensetzung im Wesentlichen durch die diTPS bestimmt wird.

Weiterhin betrifft die Erfindung ein Verfahren zur fermentativen *de novo* Synthese von Harzsäuregemischen definierter Zusammensetzung, umfassend die Kultivierung eines solchen rekombinanten Bakteriums.

Die vorliegende Erfindung betrifft die Verwendung eines solchen rekombinanten Bakteriums zur fermentativen *de novo* Synthese von Harzsäuregemischen definierter Zusammensetzung.

### Hintergrund der Erfindung/Stand der Technik

Naturharze werden schon seit dem Altertum für verschiedene Zwecke eingesetzt, unter anderem auch zum Kleben und als Dichtstoffe. Der jährliche Bedarf der chemischen Industrie in Deutschland an Naturharzen wird auf 31.000 Tonnen geschätzt. Die überwiegende Menge wird zur Herstellung von Farben, Lacken und Klebstoffen eingesetzt. Ausgehend von Naturharzen werden hierzu Harzsäuregemische aus dem Naturstoff gewonnen und für die verschiedenen Anwendungen eingesetzt, wobei die Quelle des Naturharzes die Zusammensetzung des abgeleiteten Harzsäuregemischs bestimmt, was naturgemäß saisonalen Schwankungen unterliegt.

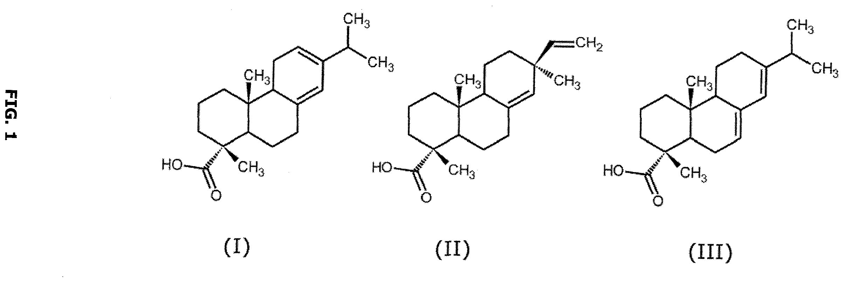

Harzsäuren bilden die Hauptbestandteile in natürlichen Harzen, wie beispielsweise dem aus Balsam (dickflüssiges, sirupartiges Gemisch aus Harzen und ätherischen Ölen) von Kiefern, Fichten und Tannen hergestellten Kolophonium. Kolophonium ist ein Sammelbegriff für aus Koniferen gewonnene, natürliche Harze, die hauptsächlich aus einem Gemisch verschiedener Harzsäuren bestehen. In dieser Form findet es allerdings technisch kaum Verwendung, da Kolophonium als Naturstoff zum einen stark schwankenden Eigenschaften unterliegt, und zum anderen nicht alterungsbeständig ist. Daher werden bislang aus Kolophonium durch Destillation die reinen Harzsäuren gewonnen, die auf verschiedene Arten weiterverarbeitet werden können. Typische Vertreter von Harzsäuren sind beispielsweise Abietinsäure und Pimarsäure (FIG. 1). Chemisch zählen die Harzsäuren zu den Carbonsäuren und gehören der Naturstoffklasse der Diterpene an.

Im Klebstoffbereich werden für Schmelzklebstoffe nahezu ausschließlich die Ester der Harzsäuren eingesetzt. Im Bereich der Lösemittelklebstoffe kommen jedoch auch überwiegend die nicht veresterten Harze zum Einsatz. Die bei der thermischen Veresterung stattfindende Umisomerisierung zum stabilsten Isomer (der Abietinsäure; FIG. 1) unterbleibt dementsprechend bei einer solchen Anwendung. Insbesondere bei der Verwendung in Lösemittelklebstoffen sind die Isomerengemische wichtig für die Funktion, da eine Reinsubstanz zu schnell und vollständig kristallisieren könnte, was negativen Einfluss auf die Hafteigenschaften haben kann.

Aus industrieller/wirtschaftlicher Sicht ist es sinnvoll und wünschenswert neue Wege zu finden, um (natürliche) Schwankungen der Produktqualität zu minimieren oder ganz zu vermeiden und um zunehmend auftretende Engpässe bei der Rohstoffversorgung zu umgehen. Darüber hinaus ist es wünschenswert, die Abhängigkeit von Rohstoffimporten zu minimieren und zugleich die Nutzung heimischer Ressourcen zu stärken. Ein geeignetes fermentatives System bietet die Aussicht, diese Punkte ideal zu verknüpfen. Eine kontrollierte und standardisierte Harzsäureproduktion könnte unter Verwendung von Zucker als Ausgangsmaterial, der aus heimischen Quellen, beispielsweise aus Zuckerrüben oder über die Verzuckerung von Lignocellulose, bereitgestellt werden könnte, erfolgen. Auch zuckerhaltige Restströme, wie sie bei verschiedensten industriellen Prozessen anfallen, könnten grundsätzlich als Ausgangsmaterial einer fermentativen Harzsäuresynthese dienen.

### Harzsäure-Biosvnthese

Gemeinsame Ausgangsverbindungen in der Biosynthese der Terpene sind das Isopentenyldiphosphat (IPP) und das durch Isomerisierung aus IPP gebildete Dimethylallyldiphosphat (DMAPP), die in der Pflanze über den cytosolischen MVA-Weg oder den in den Chloroplasten lokalisierten MEP-Syntheseweg gebildet werden (Kirby und Keasling, Annu. Rev. Plant Biol. 60:335-355 (2009)). Aus diesen Metaboliten werden die Ausgangsverbindungen der verschiedenen Terpenklassen erzeugt: Geranyldiphosphat (GPP), Farnesyldiphosphat (FPP) und Geranylgeranyldiphosphat (GGPP). Letzteres ist dabei gemeinsamer Grundstoff für die Biosynthese nicht nur der Diterpene, sondern auch der Tetraterpene (z.B. Carotinoide). Die jeweiligen Schlüsselenzyme sind die Diterpensynthase (diTPS) bzw. die Phytoensynthase für die Tetraterpene. Die diTPS formt GGPP in das zyklische Zwischenprodukt CopalylPyrophosphat (CPP) um, das dann über mehrere Oxidationsschritte zum Diterpen-Endprodukt umgesetzt wird (Lafever u.a., Arch. Biochem. Biophys. 313: 139-149 (1994)), während die Phytoensynthase zwei Moleküle GGPP zu Phytoen verknüpft. Da beide Enzyme in Chloroplasten lokalisiert sind, liegt hier eine Konkurrenzreaktion vor und die relativen Aktivitäten der Schlüsselenzyme beeinflussen/steuern den Flux aus dem vorliegenden GGPP - Pool in die beiden unterschiedlichen Stoffwechselwege.

Über die Realisierung eines synthetischen Stoffwechselwegs in geeigneten mikrobiellen Wirten könnte diese Konkurrenzreaktion vermieden werden, was, neben der prinzipbedingten Vermeidung unerwünschter Nebenprodukte, im Idealfall auch höhere Produktausbeuten verspräche. Voraussetzung hierfür ist selbstverständlich die Bereitstellung der benötigten Ausgangsverbindungen durch den Stoffwechsel des Wirtsorganismus. In bakteriellen Wirten ist der MEP-Weg in der Regel vorhanden, sodass die benötigten Grundsubstanzen IPP und DMAPP in diesen Zellen verfügbar sind. Dies gilt insbesondere auch für das häufig verwendete Bakterium *E. coli.* Allerdings verfügt *E. coli* über keine Enzymfunktionen, um das für die Diterpen-Synthese benötigte GGPP herzustellen, so dass es im Normalfall nicht bereitsteht (Toyomasu, Biosci. Biotechnol. Biochem. 72: 1168-1175 (2008)). Durch Einbau und rekombinante Expression geeigneter Enzymfunktionen sind bereits rekombinante *E*. *coli*-Stämme etabliert worden, die zur Bildung von GGPP befähigt sind. Mit ihrer Hilfe konnten bislang synthetische Stoffwechselwege zur fermentativen Produktion von Carotinoiden (Tetraterpenen) erfolgreich etabliert werden (Cunningham, Plant Cell 8: 1613-1626 (1996)).

Im Pflanzenreich sind zwei Typen von Diterpensynthasen vertreten: monofunktionale und bifunktionale Enzyme (Toyomasu, Biosci. Biotechnol. Biochem. 72: 1168-1175 (2008)). Dementsprechend wird die Bildung der Dien-Zwischenverbindungen aus denen dann die Harzsäuren erzeugt werden über zwei sukzessiv arbeitende, monofunktionale Enzyme oder ein bifunktionales Enzym katalysiert. Bifunktionale Enzymvarianten sind insbesondere für Koniferen beschrieben und charakterisiert worden (Vogel u.a., J. Biol. Chem. 271: 23262-23268 (1996)). Die Wahl der verwendeten Diterpensynthase bestimmt die synthetisierbare Harzsäure.

Die Oxidation der Diterpensynthase-Produkte zu den Harzsäuren erfolgt mittels Enzymen, die der Klasse der Cytochrom-P450-Monooxygenasen (P450-Enzyme) angehören. Hier sind bereits geeignete Enzyme beschrieben worden, die interessanterweise alle drei notwendigen Oxidationen (zum Alkohol, zum Aldehyd und dann zur Säure) katalysieren können (Ro u.a., Proc. Natl. Acad. Sci. USA 102: 8060-8065 (2005)). Die Verwertung der Dien-Zwischenverbindungen könnte wünschenswerterweise durch die Identifizierung alternativer P450-Enzyme mit besserer Substratspezifität, oder die Identifizierung und Einbindung eines zusätzlichen, Dien-spezifischen P450-Enzyms optimiert werden.

Aus technischer Sicht ist außerdem zu berücksichtigen, dass P450-Enzyme Cofaktorabhängig sind, NAD(P)H für ihre katalytische Aktivität benötigen und zumeist auf die Wechselwirkung mit externen Redoxproteinen (Cytochrom P450-Oxidoreduktasen, CPR) angewiesen sind (Jensen und Møller, Phytochemistry 71: 132-141 (2010)). Dabei konnte in den vergangenen Jahren gezeigt werden, dass die Redoxpartner einer größeren Variabilität unterliegen können als bis dato vermutet (Taylor und King, J. Chromatogr. Sci. 39: 269-272 (2001)). Es ist aber grundsätzlich möglich P450-Reduktasen erfolgreich mit unterschiedlichen P450-Enzymen zu verwenden.

Bisher ist nur die fermentative Synthese von Harzsäuren in Hefen bekannt (K. Geisler et al., Plant Physiology 171(1): 152-164 (2016); B. Hamberger et al., Plant Physiology 157(4): 1677-1695 (2011); D.-R. Ro et al., PNAS 102 (31): 8060-8065 (2005); WO 2015/197075).

Eine Aufgabe der vorliegenden Erfindung ist somit die Vereinfachung der Darstellung solcher Harzsäuren bzw. Harzsäuregemische über ein Bakterium-basiertes fermentatives Verfahren. Dieses bietet die Aussicht, je nach verwendeter Enzymkombination, unterschiedliche, Harzsäuregemische zu bilden, die je nach verwendeter Diterpensynthase eine Harzsäure als Hauptkomponente enthalten. Solche Gemische mit vorbestimmten adhäsiven und sonstigen gewünschten Eigenschaften könnten dann für den jeweiligen Anwendungszweck optimiert werden.

### Kurzbeschreibung der Erfindung

Erfindungsgemäß wurde überraschend gefunden, dass Harzsäuren und Harzsäuregemische definierter Zusammensetzung in einem Bakterium-basierten fermentativen Verfahren *de novo* synthetisiert werden können.

Dieses neuartige Verfahren erlaubt es erstmals Harzsäuren wie Abietinsäure, Pimarsäure und Levopimarsäure als einzelne Verbindungen zu erzeugen. Diese Substanzen sind in dieser Form und als einzelne Substanzen bislang am Markt nicht erhältlich. Außerdem erlaubt die Verfügbarkeit der reinen Harzsäuren durch gezielte Zumischung der fermentativ gewonnenen Präparate in vorhandene Harzsäuremischungen eine kontinuierlich hohe Qualität zu sichern.

Die vorliegende Erfindung betrifft dabei die folgenden Aspekte:
(1) ein rekombinantes Bakterium,
   das die folgenden Nukleinsäuresequenzen aufweist, die die zur fermentativen *de novo* Synthese von Harzsäuregemischen definierter Zusammensetzung erforderlichen Enzyme codieren: (i) wenigstens eine Geranylgeranyl-Pyrophosphat-Synthase (GGPPS), (ii) wenigstens eine bakterielle oder pflanzliche Isopentenyl-Pyrophosphat-Isomerase (IPI) mit für bakterielle Expression Codon-optimierter Sequenz, (iii) wenigstens eine Diterpensynthase (diTPS) mit für bakterielle Expression Codon-optimierter Sequenz, welche die Zusammensetzung bestimmt, (iv) wenigstens eine aus *Picea sitchenisis* stammende Cytochrom-P450-Monooxygenase (CYP) mit für bakterielle Expression Codon-optimierter Sequenz, und (v) wenigstens eine hybride Cytochrom-P450-Reduktase (CPR), die Proteindomänen aus *Arabidopsis* und Ingwer, eine für bakterielle Expression Codon-optimierte Sequenz und eine Sequenz, die Aminosäuresequenz mit wenigstens 50 % Identität über die gesamte Länge von SEQ ID NO:10 codiert, aufweist,
   das die Enzyme exprimiert, wobei die CPR als Fusionsprotein mit dem CYP exprimiert wird, und das die Harzsäuregemische definierter Zusammensetzung synthetisiert;
(2) ein Verfahren zur fermentativen *de novo* Synthese von Harzsäuregemischen definierter Zusammensetzung, umfassend die Kultivierung des rekombinanten Bakteriums entsprechend obigem Aspekt (1); und
(3) die Verwendung eines rekombinanten Mikroorganismus entsprechend obigem Aspekt (1) in einem Verfahren gemäß obigem Aspekt (2).

Bevorzugte Ausführungsform der Erfindung sind in den abhängigen Ansprüchen besch rieben .

### Kurzbeschreibung der Figuren

Figur 1: Chemische Strukturen der Harzsäuren. Die Abbildung zeigt die Struktur der erfindungsgemäß fermentativ herstellbaren Harzsäure Levopimarsäure (I) im Vergleich zu den verwandten Strukturen der Pimarsäure (II) und der Abietinsäure (III) .

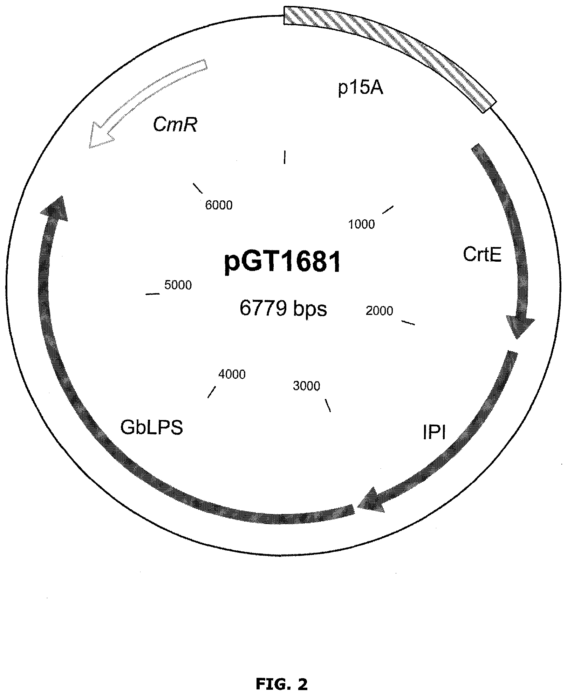

Figur 2: Plasmid enthaltend die Enzymkaskade zur Biosynthese der Grundstruktur Levopimaradien, mit crtE: Geranylgeranyl-diphosphat-Synthase; IPI: Isopentenyl-Diphosphat-Isomerase; GbLPS: Terpensynthase Levopimardiensynthase; CmR: Chloramphenicol-Resistenzgen; p15A: Regulator der Plasmidreplikation (origin of replication).

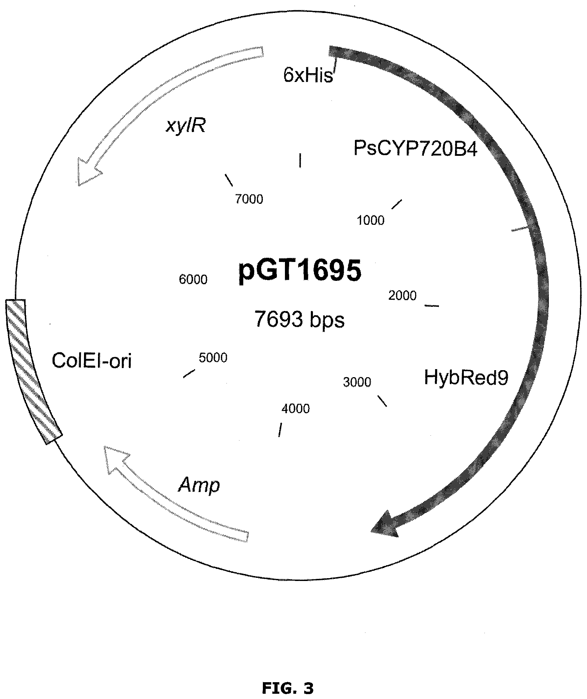

Figur 3: Plasmid enthaltend die Enzymkaskade zur oxidativen Umsetzung von Levopimaradien zu Levopimarsäure, mit 6xHis: HIS-Tag-Sequenz; PsCYP720B4: Cytochrom-P450-Monooxygenase-Domäne; HybRed9: Cytochrom-Reduktase-Domäne; Amp: Ampicillin-Resistenzgen; ColEI-ori: Regulator der Plasmidreplikation (origin of replication); xylR: Regulator des Xylosepromotors.

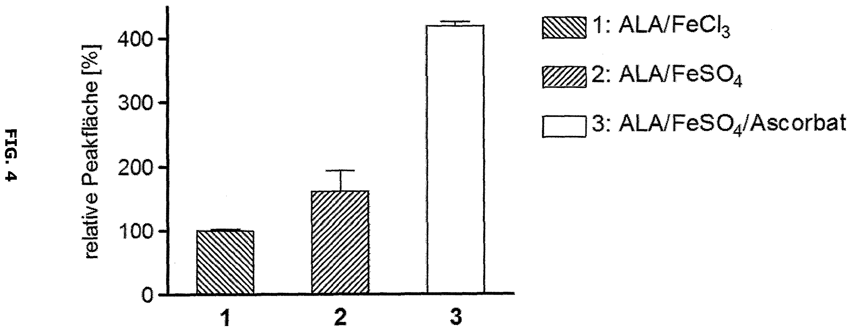

Figur 4: Medienzusatz Ascorbat bewirkt deutliche Steigerung der Produktausbeute. Eisenionen und Aminolävulinsäure sind Medienzusätze, die für eine hohe CYP-Aktivität benötigt werden. Die Verwendung von FeSO₄ (Fe²⁺) an Stelle von FeCl₃ (Fe³⁺) ist vorteilhaft (Ausbeutesteigerung: 60 %). Zusatz des Antioxidans Ascorbat bewirkt eine weitere 2,6-fache Steigerung der Ausbeute an Harzsäure.

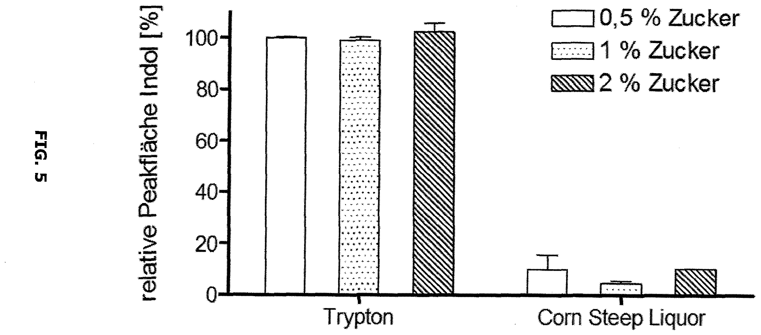

Figur 5: Minimierung des unerwünschten Stoffwechsel-Nebenprodukts Indol durch Verwendung einer geeigneten Stickstoffquelle. Unabhängig vom Gehalt der eingesetzten C-Quelle wird bei Ersatz der N-Quelle Trypton durch Corn-steep-liquor die Indolbildung um 90 bis 95 % gesenkt. Die Indol-Quantifizierung erfolgte über HPLC.

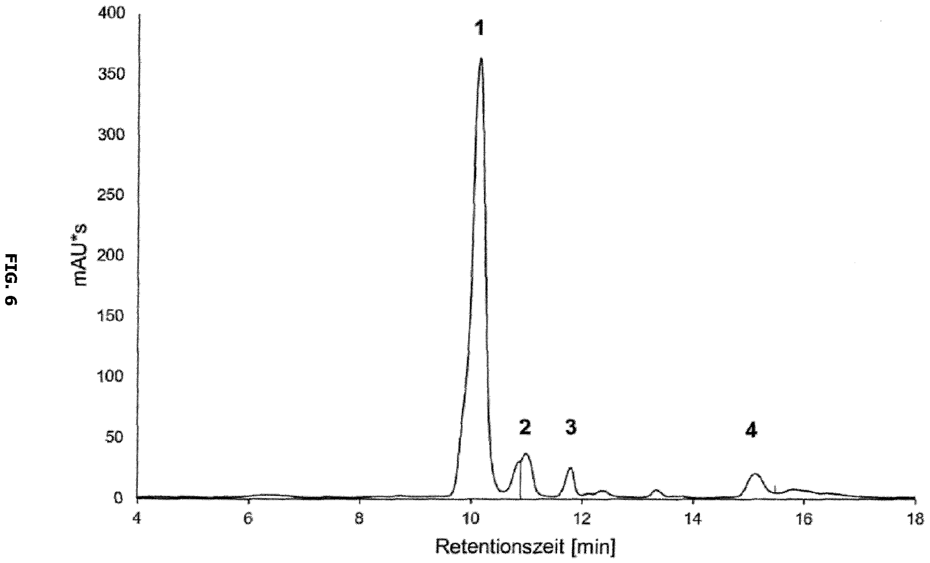

Figur 6: Typische HPLC-Analytik für erfindungsgemäß fermentativ hergestellte Levopimarsäure. Levopimarsäure wird durch sukzessive Oxidation von Levopimaradien über Levopimaradienol und Levopimaradienal generiert, wobei die Zwischenstufen zu geringen Anteilen im finalen Produkt enthalten sein können. Die Mengenanteile der oxidierten Produkte sind angegeben. 1: Levopimarsäure: 88,9 %, 2: Levopimardienal: 6,9 % 3: Levopimardienol: 4,2 %, 4: Levopimardien (Ausgangsverbindung)

### Detaillierte Beschreibung der Erfindung

Die vorliegende Erfindung betrifft das oben unter (1) beschriebenen, rekombinante Bakterium.

Unter einem "rekombinanten Bakterium" ist im Zusammenhang mit der vorliegenden Erfindung ein gentechnisch verändertes bzw. modifiziertes Bakterium zu verstehen, das in seinem Genom heterologe Nukleotidsequenzen enthält, also Nukleotidsequenzen, die so natürlicherweise nicht in dem Mikroorganismus vorkommen. Hierzu zählen insbesondere Gene eines anderen Organismus (sogenannte Transgene), aber auch im Vergleich zur natürlichen Sequenz veränderte Nukleotidsequenzen und auch Nukleotidsequenzen, die natürlicherweise an einer anderen Stelle im Genom des Mikroorganismus vorhanden sind. Diese heterologen Nukleotidsequenzen können entweder auf dem Chromosom des Bakteirums und/oder auf einem oder mehreren extrachromosomalen DNA-Elementen des Mikroorganismus vorliegen. Beispiele für extrachromosomale DNA-Elemente umfassen alle Arten von Vektoren, wie Plasmide, Cosmide, Bacterial Artificial Chromosomes (BACs), oder virale Vektoren. Bakterien im Sinne der vorliegenden Erfindung sind vorzugsweise ausgewählt aus der Gruppe bestehend aus fakultativ anaeroben Bakterien, insbesondere Proteobacteria, zum Beispiel der Gattung *Escherichia,* bevorzugt der Art *E. coli. E. coli* Stämme von besonderem Interesse im Zusammenhang mit dieser Erfindung sind *E. coli* BL21 und *E. coli* TOP10. Die gentechnische Veränderung der Bakterien kann nach jeder der im Stand der Technik bekannten biotechnologischen Verfahren erfolgen, so etwa mittels Transformation, Transduktion oder Konjugation.

Das rekombinante Bakterium weist die Enzymaktivitäten wenigstens einer Geranylgeranyl-Pyrophosphat-Synthase (GGPPS), wenigstens einer Isopentenyl-Pyrophosphat-Isomerase (IPI), wenigstens einer Diterpensynthase (diTPS), wenigstens einer Cytochrom-P450-Monooxygenase (CYP), und wenigstens einer Cytochrom-P450-Reduktase (CPR) auf. Die entsprechenden Enzyme können dabei entweder natürlicherweise in dem Mikroorganismus vorkommen oder auch nicht, also entweder endogen oder heterolog sein, wobei aber wenigstens eines der Enzyme heterolog ist. Insbesondere sind auch Kombinationen von endogenen und heterologen Enzymen von der Erfindung mitumfasst.

Eine "Geranylgeranyl-Pyrophosphat-Synthase (GGPPS)" im Sinne der vorliegenden Erfindung ist ein bakterielles Enzym, das die Synthese von Geranylgeranyl-Pyrophosphat (GGPP) aus Dimethylallylpyrophosphat (DMAPP) und Isopentenylpyrophospat (IPP) katalysiert. In einer Ausführungsform der vorliegenden Erfindung stammt die GGPPS aus Enterobakterien, besonders bevorzugt aus *Pantoea agglomerans.* Besonders bevorzugte Ausführungsformen der GGPPS sind solche, die eine Aminosäuresequenz gemäß SEQ ID NO:4 oder eine Aminosäuresequenz mit wenigstens 50 %, 60 %, 70 %, 80 %, 90 %, 95 % oder 99 % Identität über die gesamte Länge von SEQ ID NO:4 umfassen oder daraus bestehen. In einer weiteren Ausführungsform wird die GGPPS von einer Nukleotidsequenz umfassend oder bestehend aus SEQ ID NO:3 oder einer Nukleotidsequenz, die wenigstens 50 %, 60 %, 70 %, 80 %, 90 %, 95 % oder 99 % Identität über die gesamte Länge von SEQ ID NO:3 aufweist, codiert.

Die GGPP Synthese kann durch die Expression einer bakteriellen oder pflanzlichen IPP-Isomerase (IPI), welche die Isomerisierung von IPP zu DMAPP katalysiert, unterstützt werden. In einer Ausführungsform der vorliegenden Erfindung stammt die IPI aus Bakterien, bevorzugt aus Enterobakterien, besonders bevorzugt aus *Pantoea agglomerans.* Besonders bevorzugte Ausführungsformen der IPI sind solche, die eine Aminosäuresequenz gemäß SEQ ID NO:6 oder eine Aminosäuresequenz mit wenigstens 60 %, 70 %, 80 %, 90 %, 95 % oder 99 % Identität über die gesamte Länge von SEQ ID NO:6 umfassen oder daraus bestehen. In einer weiteren Ausführungsform wird die IPI von einer Nukleotidsequenz umfassend oder bestehend aus SEQ ID NO:5 oder einer Nukleotidsequenz, die wenigstens 50 %, 60 %, 70 %, 80 %, 90 %, 95 % oder 99 % Identität über die gesamte Länge von SEQ ID NO:5 aufweist, codiert. In einer weiteren Ausführungsform der vorliegenden Erfindung stammt die IPI aus Pflanzen, bevorzugt aus Zingiberales, besonders bevorzugt aus *Curcuma wenyujin* und weist eine für bakterielle Expression Codon-Optimierung auf. Besonders bevorzugte Ausführungsformen der IPI sind solche, die eine Aminosäuresequenz gemäß SEQ ID NO:14 oder eine Aminosäuresequenz mit wenigstens 80 %, 90 %, 95 % oder 99 % Identität über die gesamte Länge von SEQ ID NO:14 umfassen oder daraus bestehen. In einer weiteren Ausführungsform wird die IPI von einer Nukleotidsequenz umfassend oder bestehend aus SEQ ID NO:13 oder einer Nukleotidsequenz, die wenigstens 50 %, 60 %, 70 %, 80 %, 90 %, 95 % oder 99 % Identität über die gesamte Länge von SEQ ID NOs: 13 aufweist, codiert.

Eine "Diterpensynthase (diTPS)" im Sinne der vorliegenden Erfindung ist ein Enyzm mit für bakterielle Expression Codon-optimierter Sequenz, das GGPP in das zyklische Zwischenprodukt Copalylpyrophosphat (CPP) umwandelt, das dann über mehrere Oxidationsschritte zum Diterpen-Endprodukt umgebaut wird (Lafever u.a., Arch. Biochem. Biophys. 313: 139-149 (1994)). Eine Ausführungsform der vorliegenden Erfindung umfasst bifunktionale diTPS (bidTPS), die GGPP in zwei Schritten zum Dien zyklisieren, wobei zunächst das Zwischenprodukt Copalyldiphosphat (CPP) gebildet wird und anschließend die Modifizierung zum Dien erfolgt. Alternativ können auch monofunktionale diTPS (mdiTPS), die nur die Umwandlung von CPP zum Dien katalysieren, eingesetzt werden und zwar in Kombination mit einem zusätzlichen Enzym, einer CPP Synthase (CPS). Die Wahl der diTPS ist maßgeblich für die zu synthetisierende(n) Harzsäure(n). So erfolgt die Synthese der Levopimarsäure unter Verwendung einer Levopimardiensynthase (LPS). In einer Ausführungsform der vorliegenden Erfindung wird Levopimarsäure unter Verwendung einer pflanzlichen LPS synthetisiert, bevorzugt einer LPS aus Ginkgo, besonders bevorzugt aus *Ginkgo biloba.* Besonders bevorzugte Ausführungsformen der LPS sind solche, die eine Aminosäuresequenz gemäß SEQ ID NO:2 oder eine Aminosäuresequenz mit wenigstens 50 %, 60 %, 70 %, 80 %, 90 %, 95 % oder 99 % Identität über die gesamte Länge von SEQ ID NO:2 umfassen oder daraus bestehen. In einer weiteren Ausführungsform wird die LPS von einer Nukleotidsequenz umfassend oder bestehend aus SEQ ID NO:1 oder einer Nukleotidsequenz, die wenigstens 50 %, 60 %, 70 %, 80 %, 90 %, 95 % oder 99 % Identität über die gesamte Länge von SEQ ID NO:1 aufweist, codiert.

Die Synthese der Abietinsäure erfolgt unter Verwendung einer Abietadiensynthase (AS). In einer Ausführungsform der vorliegenden Erfindung wird Abietinsäure unter Verwendung einer pflanzlichen AS synthetisiert, bevorzugt einer AS aus Tanne, besonders bevorzugt aus *Abies grandis.* Besonders bevorzugte Ausführungsformen der AS sind solche, die eine Aminosäuresequenz gemäß SEQ ID NO:16 oder eine Aminosäuresequenz mit wenigstens 50 %, 60 %, 70 %, 80 %, 90 %, 95 % oder 99 % Identität über die gesamte Länge von SEQ ID NO:16 umfassen oder daraus bestehen. In einer weiteren Ausführungsform wird die AS von einer Nukleotidsequenz umfassend oder bestehend aus SEQ ID NO:15 oder einer Nukleotidsequenz, die wenigstens 50 %, 60 %, 70 %, 80 %, 90 %, 95 % oder 99 % Identität über die gesamte Länge von SEQ ID NO:15 aufweist, codiert.

Die Synthese der Pimarsäure/Isopimarsäure erfolgt unter Verwendung einer Pimaradien/Isopimaradiensynthase (PIM). In einer Ausführungsform der vorliegenden Erfindung werden Pimarsäure/Isopimarsäure unter Verwendung einer pflanzlichen PIM synthetisiert, bevorzugt einer PIM aus Kiefer, besonders bevorzugt aus *Pinus contorta.* Besonders bevorzugte Ausführungsformen der PIM sind solche, die eine Aminosäuresequenz gemäß SEQ ID NO:18 oder eine Aminosäuresequenz mit wenigstens 50 %, 60 %, 70 %, 80 %, 90 %, 95 % oder 99 % Identität über die gesamte Länge von SEQ ID NO:18 umfassen oder daraus bestehen. In einer weiteren Ausführungsform wird die PIM von einer Nukleotidsequenz umfassend oder bestehend aus SEQ ID NO:17 oder einer Nukleotidsequenz, die wenigstens 50 %, 60 %, 70 %, 80 %, 90 %, 95 % oder 99 % Identität über die gesamte Länge von SEQ ID NOs:17 aufweist, codiert.

Eine "Cytochrom P450 Monooxygenase (CYP)" im Sinne der vorliegenden Erfindung ist ein aus *Picea sitchensis* stammendes Enzym mit für bakterielle Expression Codon-optimierter Sequenz, das die Oxidation von Diterpen-Dienen zu Harzsäuren katalysiert. Bevorzugt sind die CYP pflanzlichen Ursprungs, insbesondere solche aus Koniferen oder dikotylen Pflanzen wie *Medicago,* Tomate oder Soja. Besonders bevorzugte Ausführungsformen der CYP sind solche aus *Picea sitchensis,* die eine Aminosäuresequenz gemäß SEQ ID NO:8 oder eine Aminosäuresequenz mit wenigstens 70 %, 80 %, 90 %, 95 % oder 99 % Identität über die gesamte Länge von SEQ ID NO:8 umfassen oder daraus bestehen. In einer weiteren Ausführungsform wird die CYP von einer Nukleotidsequenz umfassend oder bestehend aus SEQ ID NO:7 oder einer Nukleotidsequenz, die wenigstens 50 %, 60 %, 70 %, 80 %, 90 %, 95 % oder 99 % Identität über die gesamte Länge von SEQ ID NO:7 aufweist, codiert. Cytochrom-P450-Enzyme sind für die Übertragung der Elektronen auf einen Redoxpartner angewiesen. Eine Cytochrom P450 Oxidoreduktase (CPR) im Sinne der vorliegenden Erfindung ist eine CPR eine Oxidoreduktase, die als Redoxpartner der CYP dient,und die eine pflanzliche CPRs, etwa solche aus *Arabidopsis* und Ingwer.

Die CPR ist eine hybride Reduktase die durch Neukombination der Proteindomänen aus *Arabidopsis* und Ingwer erhältlich ist, eine für bakterielle Expression geeignete Codon-Optimierung aufweist und eine Aminosäuresequenz mit wenigstens 50 % Identität über die gesamte Länge von SEQ ID NO:10 aufweist, wobei die CPR als Fusionsprotein mit der CYP exprimiert wird.

Besonders bevorzugte Ausführungsformen der CPR sind solche, die eine Aminosäuresequenz gemäß SEQ ID NO:10 oder eine Aminosäuresequenz mit wenigstens 60 %, 70 %, 80 %, 90 %, 95 % oder 99 % Identität über die gesamte Länge von SEQ ID NO: 10 umfassen oder daraus bestehen. In einer weiteren Ausführungsform wird die CPR von einer Nukleotidsequenz umfassend oder bestehend aus SEQ ID NO:9 oder einer Nukleotidsequenz, die wenigstens 80 %, 90 %, 95 % oder 99 % Identität über die gesamte Länge von SEQ ID NO:10 aufweist, codiert.

Die CPR wird als Fusionsprotein zusammen mit der CYP exprimiert.

Nukleotidsequenzen, die für die vorgehend beschriebenen Enzyme codieren, können funktionell mit einem oder mehreren regulatorischen Elementen verknüpft sein. Derartige regulatorische Elemente umfassen alle im Stand der Technik bekannten regulatorischen Elemente, die die Expression des jeweiligen Enzyms beeinflussen können. Insbesondere zählen hierzu Promotoren, beispielsweise konstitutive und regulatorische Promotoren, Terminatoren, Ribosomenbindungsstellen und Enhancer. Die Expression der vorgehend beschriebenen Enzyme ist durch Codon-Optimierung der codierenden Nukleotidsequenzen, also durch Anpassung an die dem rekombinanten Bakterium eigene codon-usage, beeinflusst werden.

Die vorliegende Erfindung betrifft somit codon-optimierte Varianten der für die Enzyme codierenden Nukleotidsequenzen. So stellt beispielsweise SEQ ID NO:1 eine hinsichtlich des codon-usage von *E. coli* optimierte Variante der Levopimardiensynthase aus *Ginkgo biloba* dar.

Die vorliegende Erfindung betrifft auch das oben unter (2) beschriebene Verfahren zur fermentativen *de novo* Synthese von Harzsäuregemischen definierter Zusammensetzung, umfassend die Kultivierung eines rekombinanten Bakterium gemäß obigem Aspekt (1).

"*De novo*" Synthese bedeutet hierbei, dass die erfindungsgemäßen Endprodukte, also die Harzsäuren bzw. Harzsäuregemische, ausgehend von einfachsten Vorläufermolekülen, insbesondere Kohlenstoffverbindungen wie Zucker, gebildet werden.

"Fermentativ" meint weiterhin, dass es sich um eine enzymatische Umwandlung der Vorläufermoleküle zu den erfindungsgemäßen Endprodukten handelt.

Das erfindungsgemäße Verfahren umfasst die Kultivierung des erfindungsgemäßen rekombinanten Bakterium. Hierbei bezeichnet "Kultivierung" jede Form der Kultur, die die Synthese der erfindungsgemäßen Harzsäuren gewährleistet. Die Kultivierung erfolgt in einem geeigneten Nährmedium. Ein solches Nährmedium enthält eine Kohlenstoffquelle, bevorzugt wenigstens einen Zucker, der besonders bevorzugt ausgewählt ist aus der Gruppe umfassend Xylose, Glucose, Glycerin, Lactose, Galactose, Arabinose und Fructose, am meisten bevorzugt ist Xylose. Weiter kann das Nährmedium eine Stickstoffquelle enthalten, wobei es bevorzugt ist, dass es sich um eine Tryptophan-arme Stickstoffquelle, etwa Maisquellwasser (corn steep liquor) handelt. Weiter kann das Nährmedium Zusätze enthalten, die die Aktivität der exprimierten Enzyme beeinflussen können, beispielsweise Ascorbat oder Eisenverbindungen. Die Temperatur, bei der die Kultivierung erfolgt, richtet sich grundsätzlich nach dem zu kultivierenden Mikroorganismus. Weiter ist der Einfluss der Temperatur auf die jeweiligen Enzymaktivitäten zu berücksichtigen. In einer bevorzugten Ausführungsform der vorliegenden Erfindung wird *E. coli* bei einer Temperatur von 26 °C kultiviert.

Das erfindungsgemäße Verfahren dient der Herstellung von Harzsäuregemischen definierter Zusammensetzung. Grundsätzlich ist das Verfahren für die Herstellung einer Vielzahl verschiedener Harzsäuregemische mit unterschiedlicher, definierter Zusammensetzung, geeignet. So ist das Verfahren etwa geeignet, Gemische umfassend und/oder im Wesentlichen bestehend aus Levopimarsäure zu erzeugen. Bevorzugt ist die Herstellung von Levopimarsäure bzw. eines Gemisches, das im Wesentlichen aus Levopimarsäure besteht. Wie oben dargestellt, bestimmt die Wahl der eingesetzten Terpensynthase die herstellbaren Produkte. Die Produktion von Levopimarsäure bzw. eines Gemisches, das im Wesentlichen aus Levopimarsäure besteht, gelingt durch den Einsatz einer Levopimardiensynthase, bevorzugt einer Levopimardiensynthase aus Ginkgo. Unter einem Gemisch, das im Wesentlichen aus Levopimarsäure besteht, ist in einer Ausführungsform der vorliegenden Erfindung ein Gemisch zu verstehen, das wenigstens 80 %, bevorzugt wenigstens 82 %, besonders bevorzugt zwischen 80 % und 95 %, ganz besonders bevorzugt zwischen 82 % und 90 % Levopimarsäure enthält, wobei sich die vorstehenden Prozentangaben auf die oxidierten Levopimardienprodukte und auf eine Bestimmung mittels HPLC Analytik, wie unten beispielhaft dargestellt, beziehen.

Die Erfindung wird in dem nachfolgenden Beispiel näher erläutert:

### Beispiel

### Fermentative de novo Synthese von Levopimarsäure

Verwendeter Bakterienstamm/Wirt
*E. coli* BL21 (DE3) gold
Genotyp: F⁻ *ompT gal dem Ion hsdS_{B}(r_{B}⁻m_{B}⁻)* λ(DE3 [*lacI lacUV5-T7p07 ind1 sam7 nin5])* [*malB***⁺**]_{K-12}(λ^{S})

### Eingesetzte Plasmide

### pGT1681

Das Plasmid pGT1681, wie in FIG. 2 dargestellt, enthält die Enzymkaskade zur Biosynthese der Grundstruktur Levopimaradien.

Die benötigten Enzymfunktionen umfassen:
- eine Geranylgeranyl-diphosphat-Synthase (crtE)
- eine Isopentenyl-Diphosphat-Isomerase (IPI)
- die Terpensynthase Levopimardiensynthase (LPS)

Basierend auf dem Plasmid pAC-BETAipi (Cunninhgam and Gantt, Photosynth. Res. 92:245-259 (2007)) wurde ein artifizielles Operon erzeugt, das diese drei Enzyme kodiert. Hierzu wurde das vorhandene crtE-Gen gegen ein alternatives Gen aus *P.agglomerans* ausgetauscht, das im Experiment eine höhere Produktausbeute bewirkte. Zudem wurden die Gene crtY, crtI und crtB entfernt und durch das Gen GbLPS ersetzt. Das GbLPS-Gen stammt aus der Pflanze *Ginkgo biloba* und kodiert für eine plastidär lokalisierte Levopimardiensynthase. Für die Verwendung im fermentativen System wurden die für das Chloroplasten-Lokalisationssignal kodierenden Sequenzen entfernt und nur die für das reife Protein kodierende Sequenz in das Plasmid pGT1681 eingesetzt. Zudem wurde die DNA-Sequenz von GbLPS hinsichtlich des Codon-usage für die Expression in *E. coli* optimiert.

Experimentell wurde gefunden, dass eine Variante der verkürzten GbLPS, die die Punktmutationen M534I und Y608F trägt, 10x aktiver als das Wildtyp-Enzym ist. Die Nukleotidsequenz der in pGT1681 enthaltenen GbLPS ist in SEQ ID NO:1 gezeigt.

In der Folge sind weitere Verbesserungen des erhaltenen Plasmids vorgenommen worden, die die Produktsynthese optimieren. So wurde die intergenische Region zwischen den Genen IPI und GbLPS angepasst. Außerdem wurden die *Shine-Dalgarno*-Sequenzen vor crtE und IPI optimiert. Der artifizielle Terminator aTerm5 wurde eingefügt. Die vollständige Nukleotidsequenz von pGT1681 ist in SEQ ID NO: 12 zu finden.

### pGT1695

Das Plasmid pGT1695, wie in FIG. 3 dargestellt, enthält die Enzymkaskade zur oxidativen Umsetzung von Levopimaradien zu Levopimarsäure Die benötigten Enzymfunktionen umfassen:
- eine Cytochrom-P450-Monooxygenase (CYP), bevorzugt PsCYP720B4 (SEQ ID NOs:7, 8)
- eine Cytochrom-Reduktase (CPR), bevorzugt HybRed9 (SEQ ID NOs: 9, 10) Dieser Expressionsvektor codiert für ein Fusionsprotein aus CYP und CPR, wobei das Fusionsgen unter der Kontrolle des Xylose-induzierbaren Promotors pXYL1 steht.

Als CYP-Komponente wurde das Gen für die Monooxygenase CYP720B4 aus *Picea sitchensis eingesetzt.* Die Gensequenz ist verkürzt, so dass ein N-terminal um 29 Aminosäuren verkürztes Protein kodiert wird (Deletion der Transmembranhelix am N-Terminus). Das verwendete Gen ist codon-optimiert für die Expression in *E.coli* und seine Nukleotidsequenz in SEQ ID NO:7 gezeigt. Als CPR-Komponente für das Fusionsprotein wird das Gen HybRed9 (SEQ ID NO:9) verwendet, um die Produktausbeute zu optimieren.

Die vollständige Nukleotidsequenz von pGT1695 ist in SEQ ID NO:11 zu finden.

Die Enzymkaskaden der Plasmide pGT1681 und pGT1695 können alternativ auch in das Genom des Wirtsstammes integriert werden. Auf selektive Antibiotika kann in diesem Fall bei der fermentativen Produktion (s.u.) verzichtet werden.

### Kulturmedien und Kultivierung/Fermentation

Die Medien und Kulturbedingungen sind für die Harzsäureproduktion mit dem genannten Mikroorganismus optimiert worden. Die Effekte der Verbesserungen sind in der Folge dargestellt:
- Der Stamm BL21 synthetisiert 8x mehr Levopimaradien und 3x mehr Levopimarsäure als der ursprünglich verwendete Stamm *E.coli* TOP10.
- Anpassung der Kultur-Temperatur auf 26 °C bewirkt verbesserte Oxidaseaktivität.
- Zugabe der Hämvorstufe Aminolävulinsäure (ALA) und FeCl₃ zum Medium mit positiver Wirkung auf die Ausbeute an aktivem CYP-Enzym. Resultiert in höherer Oxidaseaktivität und besserer Endproduktbildung.
- Ersatz von FeCl₃ durch FeSO₄ bewirkt 60 %-ig höhere Produktausbeute (siehe FIG. 4).
- Verwendung von Ascorbat als Medienzusatz bewirkt eine weitere 2,6-fache Steigerung der Produktausbeute (siehe FIG. 4).
- Verwendung von Corn Steep Liquor statt Trypton als Aminosäure/Stickstoffquelle bewirkt eine 90 %-ige Senkung der Indol-Nebenproduktion (siehe FIG. 5).

### Medien:

dYT -Medium
   1,6 g/l Stickstoffquelle*
   1,0 g/l Hefeextrakt
   0.5 g/l NaCl
   (+ 15 g/l Agar bei festen Medien)
   pH 7,0 → autoklaviert
*Stickstoffquelle können Trypton, Corn steep liquor (CSL), Beef-Extract oder Ammoniumsulfat bzw. Ammoniumchlorid sein. Die Ammoniumsalze oder CSL werden bevorzugt.

### Antibiotika

100 mg/l Ampicillin
25 mg/l Chloramphenicol

### Medienzusätze

| Endkonzentration im Medium | |
|---|---|
| 1x | Makro-Elemente* |
| 250 µM | Aminolävulinsäure |
| 25 µM | FeCl₃ oder FeSO₄ |
| 10 mM | Ascorbat |
| 30 g/l | Xylose |

| | |
|---|---|
| *10x-Makro-Elemente: 98 g/l K₂HPO₄ 3 g/l Ammonium iron (III) citrate (Sigma F5879-100G) 21 g/l Citric acid Monohydrate → sterilfiltriert | |

### Durchführung der Fermentation

Zur Kultivierung der Harzsäure-produzierenden Bakterienkulturen im Labormaßstab werden die Plasmide pGT1681 und pGT1695 in chemisch kompetente Zellen von *E*. *coli* BL21 mittels Hitzeschock transformiert wie es dem Fachmann bekannt ist. Zur Selektion positiver Transformanten wird die Kultur auf dYT-Medium (fest) mit Antibiotika (Amp + Cm) ausplattiert und die Platte über Nacht bei 30 °C inkubiert. Für die fermentative Produktion der Harzsäuren im Schüttelkolben werden zunächst im Reagenzglas Vorkulturen angeimpft. Hierzu werden 3 ml dYT-Medium (flüssig) mit Antibiotika mit jeweils einer Kolonie beimpft und für ca. 4 Stunden bis zu einer Zelldichte von OD600≈0,6-1 bei 30 °C und 200 Umdrehungen/Minute (rpm), sodass ausreichender Sauerstofftransfer in das Nährmedium gewährleistet ist, angezogen. Für die Hauptkultur werden 15 ml auf 26 °C vorgewärmtes dYT-Medium (flüssig) mit Zusätzen und Antibiotika im 100 ml-Erlenmeyerkolben mit entsprechendem Volumen Vorkultur beimpft, sodass eine Zelldichte von OD600=0,033 vorliegt. Nach 20 h Kultivierung bei 26 °C und 200 Umdrehungen/Minute werden weitere 30 g/l Xylose zugegeben, die Kultur für weitere 20 Stunden bei 26 °C und 200 Umdrehungen/Minute inkubiert. Danach erfolgt die Abtrennung der Produkt-haltigen Biomasse vom Nährmedium mittels Zentrifugation (20 min, 5000 x g).

Produktion im größeren Volumen kann alternativ im Fermenter (Fed-batch Fermentation) erfolgen. Die benötigte Xylose wird hierbei über den Feed dem Fermenter zu geführt. Xylose dient zugleich als Kohlenstoffquelle und Induktor für die Expression des Oxidasesystems.

### Harzsäure-Extraktion zur Analytik

Material:
Saline: 0,9 % NaCl
Aceton (p.a.)
n-Hexan (p.a.)
Acetonitril (gradient grade)

Um Harzsäuren, Aldehyd, Alkohol und Olefine aus der Expressionskultur zu extrahieren wird diese in ein 50 ml-Zentrifugengefäß überführt und 5 Minuten bei 400 Umdrehungen/Minute abzentrifugiert. Der Überstand enthält keine signifikanten Mengen an Harzsäuren und wird daher verworfen. Das Zellpellet wird in 400 µl 0,9 % NaCl (Saline) resuspendiert und in ein 2 ml-Zentrifugengefäß überführt. Zum Zellaufschluss werden 400 µl Aceton (p.a.) zugegeben und gut gemischt bis das Zellpellet vollständig resuspendiert ist. Folgende Schritte werden insgesamt dreimal durchgeführt. Zur Extraktion werden 400 µl n-Hexan zugegeben, gut gemischt und 10 Minuten geschüttelt, anschließend 20-25 Sekunden bei 14.000 Umdrehungen/Minute abzentrifugiert. Der organische Lösungsmittelüberstand wird abpipettiert und in einem 1,5 ml Zentrifugengefäß gesammelt. Dieser enthält die Harzsäuren und ihre Vorstufen. Bei den Ausschüttel-Schritten wird darauf geachtet, dass das Zellpellet vollständig resuspendiert wird.

### Alle Arbeiten erfolgen bei Raumtemperatur

Nach mehreren Optimierungsschritten und Versuchen u.a. mit Ethylacetat und Acetonitril als Lösungsmittel, hat sich gezeigt, dass durch Kombination von Zellaufschluss mit Aceton und Extraktion mit n-Hexan die Harzsäuren und ihre Vorstufen am effizientesten extrahiert werden können. Die Arbeitszeit wurde deutlich verkürzt und die Extraktion der Produkte erfolgt quantitativ, sodass die verbleibenden Reststoffe keine signifikanten Produktmengen mehr enthalten.

### HPLC-Analyse

Vorbereitend zur Analyse der erhaltenen Harzsäureprodukte wird der erzeugte n-Hexan-Extrakt in einer Vakuumzentrifuge bei 2000 Umdrehungen/Minute und 40 °C unter Vakuum für 30 Minuten bis zur Trockne eingedampft. Der Rückstand wird in 200 µl Acetonitril (gradient grade) gelöst und für die HPLC-Analytik über Spartan^{®}-Spritzenvorsatzfilter (0,2 µm Porengröße) vorgereinigt, um feinste Feststoffverunreinigungen zu entfernen.

Der Nachweis der Harzsäuren und ihrer Vorstufen erfolgt mittels HPLC. Die Trennung erfolgt über einen Gradienten von 20 % bis 100 % Laufmittel B in 10 Minuten mit Laufmittel A: 20 % Acetonitril in Wasser + 0,25 % Essigsäure und Laufmittel B: 100 % Acetonitril + 0,25 % Essigsäure bei einer Flussrate von 1 ml/Minute über eine Purospher^{®}Star PR 18e 5 µm LiChroCART^{®} 125-4 Säule. Die Detektion erfolgt für Levopimaradien-Derivate bei 270 nm, für Abietadien-/Pimaradien-Derivate bei 240 nm.

Die Ergebnisse zeigen, dass bei Durchführung des erfindungsgemäßen fermentativen Verfahrens mit dem Enzym Levopimardiensynthase (LPS) die Levopimarsäure das Hauptprodukt darstellt. Dessen Anteil an den oxidierten Levopimardienprodukten beträgt typischerweise 82-90 %. Daneben treten die unmittelbaren Vorstufen geringerer Oxidationsstufe (Aldehyd (-dienal) bzw. Alkohol(-dienol)) mit Anteilen von jeweils 5-9 % im Produktprofil auf. Ein typisches Chromatogramm ist in FIG. 6 dargestellt.

## Patentansprüche

1. Rekombinantes Bakterium, das die folgende Nukleinsäuresequenzen aufweist, die die zur fermentativen *de novo* Synthese von Harzsäuregemischen definierter Zusammensetzung erforderlichen Enzyme codieren:
(i) wenigstens eine bakterielle Geranylgeranyl-Pyrophosphat-Synthase (GGPPS),
(ii) wenigstens eine bakterielle oder pflanzliche Isopentenyl-Pyrophosphat-Isomerase (IPI) mit für bakterielle Expression Codon-optimierter Sequenz,
(iii) wenigstens eine Diterpensynthase (diTPS) mit für bakterielle Expression Codon-optimierter Sequenz, welche die Zusammensetzung bestimmt,
(iv) wenigstens eine aus *Picea sitchensis* stammende Cytochrom-P450-Monooxygenase (CYP) mit für bakterielle Expression Codon-optimierter Sequenz, und
(v) wenigstens eine hybride Cytochrom-P450-Reduktase (CPR), die Proteindomänen aus *Arabidopsis* und Ingwer, eine für bakterielle Expression Codon-optimierte Sequenz und eine Sequenz, die eine Aminosäuresequenz mit wenigstens 50 % Identität über die gesamte Länge von SEQ ID NO:10 codiert, aufweist,
das die Enzyme exprimiert, wobei die CPR als Fusionsprotein mit dem CYP exprimiert wird, und das die Harzsäuregemische definierter Zusammensetzung synthetisiert.

2. Rekombinantes Bakterium nach Anspruch 1, **dadurch gekennzeichnet, dass**
(i) das Harzsäuregemisch im Wesentlichen aus Levopimarsäure besteht und die diTPS eine Levopimardiensynthase ist; oder
(ii) das Harzsäuregemisch im Wesentlichen aus Abietinsäure besteht und die diTPS eine Abietadiensynthase ist; oder
(iii) das Harzsäuregemisch im Wesentlichen aus Pimarsäure/Isopimarsäure besteht und die diTPS eine Pimaradien/Isopimaradiensynthase ist.

3. Rekombinantes Bakterium nach Anspruch 2, **dadurch gekennzeichnet, dass**
(i) die diTPS eine Aminosäuresequenz mit wenigstens 50 % Identität über die gesamte Länge von SEQ ID NO:2 umfasst, bevorzugt SEQ ID NO:2 umfasst; oder
(ii) die diTPS eine Aminosäuresequenz mit wenigstens 50 % Identität über die gesamte Länge von SEQ ID NO:16 umfasst, bevorzugt SEQ ID NO:16 umfasst; oder
(iii) die diTPS eine Aminosäuresequenz mit wenigstens 50 % Identität über die gesamte Länge von SEQ ID NO:18 umfasst, bevorzugt SEQ ID NO:18 umfasst.

4. Rekombinantes Bakterium nach Anspruch 2 oder 3, **dadurch gekennzeichnet, dass**
(i) die diTPS von einer Nukleotidsequenz gemäß SEQ ID NO:1 oder einer Nukleotidsequenz mit wenigstens 50 % Identität über die gesamte Länge von SEQ ID NO:1 codiert wird; oder
(ii) die diTPS von einer Nukleotidsequenz gemäß SEQ ID NO:15 oder einer Nukleotidsequenz mit wenigstens 50 % Identität über die gesamte Länge von SEQ ID NO:15 codiert wird; oder
(iii) die diTPS von einer Nukleotidsequenz gemäß SEQ ID NO:17 oder einer Nukleotidsequenz mit wenigstens 50 % Identität über die gesamte Länge von SEQ ID NO:17 codiert wird.

5. Rekombinantes Bakterium nach einem der Ansprüche 1 bis 4, **dadurch gekennzeichnet, dass**
(i) die GGPPS aus Enterobakterien, bevorzugt aus *Pantoea agglomerans* stammt; und/oder
(ii) die IPI aus Enterobakterien, bevorzugt aus *Pantoea agglomerans,* stammt oder die IPI aus Zingiberales, bevorzugt aus *Curcuma wenyujin* stammt.

6. Rekombinantes Bakterium nach einem der Ansprüche 1 bis 5, **dadurch gekennzeichnet, dass**
(i) die GGPPS eine Aminosäuresequenz mit wenigstens 50 % Identität über die gesamte Länge von SEQ ID NO:4 umfasst, bevorzugt die Aminosäuresequenz von SEQ ID NO:4 umfasst; und/oder
(ii) die IPI eine Aminosäuresequenz mit wenigstens 60 % Identität über die gesamte Länge von SEQ ID NO:6 oder 80 % Identität zu SEQ ID NO:14 umfasst, bevorzugt die Aminosäuresequenz von SEQ ID NO:6 oder SEQ ID NO:14 umfasst; und/oder
(iii) die CYP eine Aminosäuresequenz mit wenigstens 70 % Identität über die gesamte Länge von SEQ ID NO:8 umfasst, bevorzugt die Aminosäuresequenz von SEQ ID NO:8 umfasst; und/oder
(iv) die CPR eine Aminosäuresequenz mit wenigstens 80 % Identität über die gesamte Länge von SEQ ID NO:10 umfasst, bevorzugt SEQ ID NO:10 umfasst.

7. Rekombinantes Bakterium nach einem der Ansprüche 1 bis 6, **dadurch gekennzeichnet, dass**
(i) die GGPPS von einer Nukleotidsequenz gemäß SEQ ID NO:3 oder einer Nukleotidsequenz mit wenigstens 40 % Identität über die gesamte Länge von SEQ ID NO:3 codiert wird; und/oder
(ii) die IPI von einer Nukleotidsequenz gemäß SEQ ID NO:5 oder SEQ ID NO:13 oder einer Nukleotidsequenz mit wenigstens 40 % Identität über die gesamte Länge von SEQ ID NO:5 oder SEQ ID NO:13 codiert wird; und/oder
(iii) die CYP von einer Nukleotidsequenz gemäß SEQ ID NO:7 oder einer Nukleotidsequenz mit wenigstens 40 % Identität über die gesamte Länge von SEQ ID NO:7 codiert wird; und/oder
(iv) die CPR von einer Nukleotidsequenz gemäß SEQ ID NO:9 oder einer Nukleotidsequenz mit wenigstens 40 % Identität über die gesamte Länge von SEQ ID NO:9 codiert wird.

8. Rekombinantes Bakterium nach einem der Ansprüche 1 bis 7, **dadurch gekennzeichnet, dass** wenigstens eines der Enzyme von einem extrachromosomalen DNA Element codiert wird, bevorzugt von einem Plasmid.

9. Rekombinantes Bakterium nach einem der Ansprüche 1, 4 oder 7, **dadurch gekennzeichnet, dass** die Nukleotidsequenz funktionell mit wenigstens einem regulatorischen Element verknüpft ist, bevorzugt ausgewählt aus der Gruppe bestehend aus einem Promoter, einem Terminator, einem Enhancer, einer Ribosomenbindungsstelle.

10. Rekombinantes Bakterium nach einem der Ansprüche 1 bis 9, wobei das Bakterium aus der Gattung *Escherichia* ist.

11. Rekombinantes Bakterium nach Anspruch 10, das ein *E. coli* Stamm ist.

12. Verfahren zur fermentativen *de novo* Synthese von Harzsäuregemischen definierter Zusammensetzung, umfassend die Kultivierung eines rekombinanten Bakteriums nach einem der Ansprüche 1 bis 11.

13. Verfahren nach Anspruch 12, **dadurch gekennzeichnet, dass** die Kultivierung in einem Nährmedium erfolgt, das eine Kohlenstoffquelle, bevorzugt Zucker, besonders bevorzugt Xylose, Glucose, Glycerin, Lactose, Galactose, Arabinose oder Fructose, enthält.

14. Verfahren nach Anspruch 13, **dadurch gekennzeichnet, dass** das Nährmedium eine Stickstoffquelle, bevorzugt eine Tryptophan-arme Stickstoffquelle, besonders bevorzugt Maisquellwasser enthält.

15. Verwendung eines rekombinanten Bakteriums nach einem der Ansprüche 1 bis 11 zur fermentativen *de novo* Synthese von Harzsäuren sowie Harzsäuregemischen definierter Zusammensetzung.

## Claims

1. A recombinant bacterium having the following nucleic acid sequences, which code for the enzymes required for the fermentative de-novo synthesis of mixtures of resin acids having a defined composition:
(i) at least one bacterial geranylgeranyl pyrophosphate synthase (GGPPS),
(ii) at least one bacterial or plant-derived isopentenyl pyrophosphate isomerase (IPI) having a sequence that is codon-optimized for bacterial expression,
(iii) at least one diterpene synthase (diTPS) having a sequence that is codon-optimized for bacterial expression, and determining the composition,
(iv) at least one cytochrome P450 monooxygenase (CYP) derived from *Picea sitchensis* and having a sequence that is codon-optimized for bacterial expression, and
(v) at least one hybrid cytochrome P450 reductase (CPR) having protein domains from *Arabidopsis* and ginger, a sequence that is codon-optimized for bacterial expression, and a sequence that codes for an amino acid sequence having at least 50% identity over the entire length of SEQ ID NO: 10,
which expresses the enzymes, wherein said CPR is expressed as a fusion protein with CYP, and which synthesizes said mixtures of resin acids having a defined composition.

2. The recombinant bacterium according to claim 1, **characterized in that**
(i) said mixture of resin acids essentially consists of levopimaric acid, and said diTPS is a levopimaradiene synthase; or
(ii) said mixture of resin acids essentially consists of abietic acid, and said diTPS is an abietadiene synthase; or
(iii) said mixture of resin acids essentially consists of pimaric acid/iso-pimaric acid, and said diTPS is a pimaradiene/isopimaradiene synthase.

3. The recombinant bacterium according to claim 2, **characterized in that**
(i) said diTPS includes an amino acid sequence having at least 50% identity over the entire length of SEQ ID NO: 2, preferably includes SEQ ID NO: 2; or
(ii) said diTPS includes an amino acid sequence having at least 50% identity over the entire length of SEQ ID NO: 16, preferably includes SEQ ID NO: 16; or
(iii) said diTPS includes an amino acid sequence having at least 50% identity over the entire length of SEQ ID NO: 18, preferably includes SEQ ID NO: 18.

4. The recombinant bacterium according to claim 2 or 3, **characterized in that**
(i) said diTPS is encoded by a nucleotide sequence according to SEQ ID NO: 1, or a nucleotide sequence having at least 50% identity over the entire length of SEQ ID NO: 1; or
(ii) said diTPS is encoded by a nucleotide sequence according to SEQ ID NO: 15, or a nucleotide sequence having at least 50% identity over the entire length of SEQ ID NO: 15; or
(iii) said diTPS is encoded by a nucleotide sequence according to SEQ ID NO: 17, or a nucleotide sequence having at least 50% identity over the entire length of SEQ ID NO: 17.

5. The recombinant bacterium according to any of claims 1 to 4, **characterized in that**
(i) said GGPPS is derived from Enterobacteriaceae, preferably from *Pantoea agglomerans*; and/or
(ii) said IPI is derived from Enterobacteriaceae, preferably from *Pantoea agglomerans*; or said IPI is derived from Zingiberales, preferably from *Curcuma wenyujin.*

6. The recombinant bacterium according to any of claims 1 to 5, **characterized in that**
(i) said GGPPS includes an amino acid sequence having at least 50% identity over the entire length of SEQ ID NO: 4, preferably includes the amino acid sequence of SEQ ID NO: 4; and/or
(ii) said IPI includes an amino acid sequence having at least 60% identity over the entire length of SEQ ID NO: 6, or 80% identity with SEQ ID NO: 14, preferably includes the amino acid sequence of SEQ ID NO: 6 or SEQ ID NO: 14; and/or
(iii) said CYP includes an amino acid sequence having at least 70% identity over the entire length of SEQ ID NO: 8, preferably includes the amino acid sequence of SEQ ID NO: 8; and/or
(iv) said CPR includes an amino acid sequence having at least 80% identity over the entire length of SEQ ID NO: 10, preferably includes SEQ ID NO: 10.

7. The recombinant bacterium according to any of claims 1 to 6, **characterized in that**
(i) said GGPPS is encoded by a nucleotide sequence according to SEQ ID NO: 3, or a nucleotide sequence having at least 40% identity over the entire length of SEQ ID NO: 3; and/or
(ii) said IPI is encoded by a nucleotide sequence according to SEQ ID NO: 5 or SEQ ID NO: 13, or a nucleotide sequence having at least 40% identity over the entire length of SEQ ID NO: 5 or SEQ ID NO: 13; and/or
(iii) said CYP is encoded by a nucleotide sequence according to SEQ ID NO: 7, or a nucleotide sequence having at least 40% identity over the entire length of SEQ ID NO: 7; and/or
(iv) said CPR is encoded by a nucleotide sequence according to SEQ ID NO: 9, or a nucleotide sequence having at least 40% identity over the entire length of SEQ ID NO: 9.

8. The recombinant bacterium according to any of claims 1 to 7, **characterized in that** at least one of the enzymes is encoded by an extrachromosomal DNA element, preferably by a plasmid.

9. The recombinant bacterium according to any of claims 1, 4 or 7, **characterized in that** said nucleotide sequence is functionally linked with at least one regulatory element, which is preferably selected from the group consisting of a promoter, a terminator, an enhancer, a ribosome binding site.

10. The recombinant bacterium according to any of claims 1 to 9, wherein said bacterium belongs to the genus *Escherichia.*

11. The recombinant bacterium according to claim 10, which is an *E. coli* strain.

12. A process for the fermentative de-novo synthesis of mixtures of resin acids having a defined composition, comprising culturing a recombinant bacterium according to any of claims 1 to 11.

13. The process according to claim 12, **characterized in that** said culturing is effected in a nutrient medium containing a carbon source, preferably a sugar, more preferably xylose, glucose, glycerol, lactose, galactose, arabinose, or fructose.

14. The process according to claim 13, **characterized in that** said nutrient medium contains a nitrogen source, preferably a low tryptophan nitrogen source, more preferably corn steep liquor.

15. Use of a recombinant bacterium according to any of claims 1 to 11 for the fermentative de-novo synthesis of resin acids and mixtures of resin acids having a defined composition.

## Revendications

1. Bactérie recombinante présentant les séquences d'acide nucléique ci-dessous qui codent pour les enzymes nécessaires à la synthèse fermentative *de novo* de mélanges à base d'acide résinique de composition définie :
(i) au moins une géranylgéranyl-pyrophosphate-synthase (GGPPS) bactérienne,
(ii) au moins une isopentényl-pyrophosphate-isomérase (IPI) bactérienne ou végétale comportant une séquence à codons optimisés pour l'expression bactérienne,
(iii) au moins une diterpène-synthase (diTPS) comportant une séquence à codons optimisés pour l'expression bactérienne, qui détermine la composition,
(iv) au moins une cytochrome-P450-monooxygénase (CYP) provenant de *Picea sitchensis* et comportant une séquence à codons optimisés pour l'expression bactérienne, et
(v) au moins une cytochrome-P450-réductase (CPR) hybride présentant des domaines protéiques issus d'*Arabidopsis* et du gingembre, une séquence à codons optimisés pour l'expression bactérienne et une séquence codant pour une séquence d'acides aminés ayant au moins 50 % d'identité sur toute la longueur de la SEQ ID NO :10,
qui exprime les enzymes, la CPR étant exprimée sous forme de protéine de fusion avec la CYP, et qui synthétise les mélanges à base d'acide résinique de composition définie.

2. Bactérie recombinante selon la revendication 1, **caractérisée en ce que**
(i) le mélange à base d'acide résinique se compose essentiellement d'acide lévopimarique et la diTPS est une lévopimaradiène-synthase ; ou
(ii) le mélange à base d'acide résinique se compose essentiellement d'acide abiétique et la diTPS est une abiétadiène-synthase ; ou
(iii) le mélange à base d'acide résinique se compose essentiellement d'acide pimarique/d'acide isopimarique et la diTPS est une pimaradiène/isopimaradiène-synthase.

3. Bactérie recombinante selon la revendication 2, **caractérisée en ce que**
(i) la diTPS comprend une séquence d'acides aminés ayant au moins 50 % d'identité sur toute la longueur de la SEQ ID NO :2, de manière préférée comprend la SEQ ID NO :2 ; ou
(ii) la diTPS comprend une séquence d'acides aminés ayant au moins 50 % d'identité sur toute la longueur de la SEQ ID NO :16, de manière préférée comprend la SEQ ID NO :16 ; ou
(iii) la diTPS comprend une séquence d'acides aminés ayant au moins 50 % d'identité sur toute la longueur de la SEQ ID NO :18, de manière préférée comprend la SEQ ID NO :18.

4. Bactérie recombinante selon la revendication 2 ou 3, **caractérisée** ce que
(i) la diTPS est codée par une séquence de nucléotides selon la SEQ ID NO :1 ou par une séquence de nucléotides ayant au moins 50 % d'identité sur toute la longueur de la SEQ ID NO :1 ; ou
(ii) la diTPS est codée par une séquence de nucléotides selon la SEQ ID NO :15 ou par une séquence de nucléotides ayant au moins 50 % d'identité sur toute la longueur de la SEQ ID NO :15 ; ou
(iii) la diTPS est codée par une séquence de nucléotides selon la SEQ ID NO :17 ou par une séquence de nucléotides ayant au moins 50 % d'identité sur toute la longueur de la SEQ ID NO :17.

5. Bactérie recombinante selon l'une des revendications 1 à 4, **caractérisée en ce que**
(i) la GGPPS provient d'entérobactéries, de manière préférée de *Pantoea agglomerans* ; et/ou
(ii) l'IPI provient d'entérobactéries, de manière préférée de *Pantoea agglomerans*, ou l'IPI provient d'une Zingibérale, de manière préférée de *Curcuma* wenyujin.

6. Bactérie recombinante selon l'une des revendications 1 à 5, **caractérisée en ce que**
(i) la GGPPS comprend une séquence d'acides aminés ayant au moins 50 % d'identité sur toute la longueur de la SEQ ID NO :4, de manière préférée comprend la séquence d'acides aminés de la SEQ ID NO :4 ; et/ou
(ii) l'IPI comprend une séquence d'acides aminés ayant au moins 60 % d'identité sur toute la longueur de la SEQ ID NO :6 ou 80 % d'identité par rapport à la SEQ ID NO :14, de manière préférée comprend la séquence d'acides aminés de la SEQ ID NO :6 ou de la SEQ ID NO :14 ; et/ou
(iii) la CYP comprend une séquence d'acides aminés ayant au moins 70 % d'identité sur toute la longueur de la SEQ ID NO :8, de manière préférée comprend la séquence d'acides aminés de la SEQ ID NO :8 ; et/ou
(iv) la CPR comprend une séquence d'acides aminés ayant au moins 80 % d'identité sur toute la longueur de la SEQ ID NO :10, de manière préférée comprend la SEQ ID NO :10.

7. Bactérie recombinante selon l'une des revendications 1 à 6, **caractérisée en ce que**
(i) la GGPPS est codée par une séquence de nucléotides selon la SEQ ID NO :3 ou par une séquence de nucléotides ayant au moins 40 % d'identité sur toute la longueur de la SEQ ID NO :3 ; et/ou
(ii) l'IPI est codée par une séquence de nucléotides selon la SEQ ID NO :5 ou la SEQ ID NO :13 ou par une séquence de nucléotides ayant au moins 40 % d'identité sur toute la longueur de la SEQ ID NO :5 ou de la SEQ ID NO :13 ; et/ou
(iii) la CYP est codée par une séquence de nucléotides selon la SEQ ID NO :7 ou par une séquence de nucléotides ayant au moins 40 % d'identité sur toute la longueur de la SEQ ID NO :7 ; et/ou
(iv) la CPR est codée par une séquence de nucléotides selon la SEQ ID NO :9 ou par une séquence de nucléotides ayant au moins 40 % d'identité sur toute la longueur de la SEQ ID NO :9.

8. Bactérie recombinante selon l'une des revendications 1 à 7, **caractérisée en ce qu'**au moins une des enzymes est codée par un élément d'ADN extrachromosomique, de manière préférée par un plasmide.

9. Bactérie recombinante selon l'une des revendications 1, 4 ou 7, **caractérisée en ce que** la séquence de nucléotides est liée fonctionnellement à au moins un élément régulateur, de manière préférée choisi parmi le groupe constitué d'un promoteur, d'un terminateur, d'un activateur, d'un site de liaison du ribosome.

10. Bactérie recombinante selon l'une des revendications 1 à 9, dans laquelle la bactérie est du genre *Escherichia.*

11. Bactérie recombinante selon la revendication 10, qui est une souche d'*E*. *coli.*

12. Procédé de synthèse fermentative *de novo* de mélanges à base d'acide résinique de composition définie, comprenant la culture d'une bactérie recombinante selon l'une des revendications 1 à 11.

13. Procédé selon la revendication 12, **caractérisé en ce que** la culture a lieu dans un milieu de culture contenant une source de carbone, de manière préférée du sucre, de manière particulièrement préférée du xylose, du glucose, du glycérol, du lactose, du galactose, de l'arabinose ou du fructose.

14. Procédé selon la revendication 13, **caractérisé en ce que** le milieu de culture contient une source d'azote, de manière préférée une source d'azote pauvre en tryptophane, de manière particulièrement préférée de l'eau de trempe de maïs.

15. Utilisation d'une bactérie recombinante selon l'une des revendications 1 à 11 pour la synthèse fermentative *de novo* d'acides résiniques ainsi que de mélanges à base d'acide résinique de composition définie.
